# EUROPEAN PATENT SPECIFICATION

(11) **EP 0 859 629 B1**
(45) Date of publication and mention of the grant of the patent: **13.08.2003**
(21) Application number: 96942738.4
(22) Date of filing: 06.11.1996
(51) Int. Cl.: A61K 38/17, A61P 27/16

(54) **PHOSPHORYLATED RECOMBINANT HUMAN BETA CASEIN FOR INHIBITION OF ATTACHMENT OF H. INFLUENZAE TO HUMAN CELLS**
PHOSPHORYLIERTES REKOMBINANTES MENSCHLICHES BETA-KASEIN FÜR DIE HEMMUNG DER ANHEFTUNG VON H. INFLUENZAE AN MENSCHLICHEN ZELLEN
BETA-CASEINE HUMAINE RECOMBINANTE ET PHOSPHORYLEE POUR INHIBER LA FIXATION DE H. INFLUENZAE SUR DES CELLULES HUMAINES

(30) Priority: 06.11.1995 US 553941
(43) Date of publication of application: 26.08.1998
(73) Proprietor: Abbott Laboratories, Abbott Park, Illinois 60064-3500 (US)
(72) Inventor: MUKERJI, Pradip, Gahanna, OH 43230 (US); THURMOND, Jennifer, Marie, Columbus, OH 43231 (US); HANSSON, Lennart, S-38 Umeä (SE); BAXTER, Jennifer, Harris, Galena, OH 43201 (US); HARDS, Robert, George, Delaware, OH 43015 (US); LEONARD, Amanda, Eun-Yeong, Gahanna, OH 43230 (US); ANDERSON, Steven, Neal, Columbus, OH 43213 (US); Regan, Linda Ann, Fremont, CA 94536 (US)
(74) Representative: Modiano, Guido, Dr.-Ing.
(86) International application number: US9617971
(87) International publication number: WO97026320

(56) References cited:
- WO-A-91/06308
- WO-A-91/08675
- WO-A-96/27018
- WO-A-97/17085
- WO-A-97/17449
- ANIANSSON G. ET AL.: "ANTI-ADHESIVE ACTIVITY OF HUMAN CASEIN AGAINST STREPTOCOCCUS PNEUMONIAE AND HAEMOPHILUS INFLUENZAE", MICROBIAL PATHOGENESIS, , 1990, vol. 8, no. , pages 315 to 323
- GREENBERG R. ET AL.: "HUMAN BETA-CASEIN", JOURNAL OF BIOLOGICAL CHEMISTRY, , 25. April 1984, vol. 256, no. 8, pages 5132 to 5136

### SEQUENCE LISTING

(1) GENERAL INFORMATION:
   (i) APPLICANTS:
      Mukerji, P.
      Thurmond, J.
      Hansson, L
      Baxter, J.
      Hards, R.
      Leonard, A.
      Anderson, S.
      Harvey, L.
   (ii) TITLE OF INVENTION: Product for Inhibition of Attachment of H. Influenzae to Human Cells
   (iii) NUMBER OF SEQUENCES: 4
   (iv) CORRESPONDENCEADDRESS:
      (A) ADDRESSEE: Donald O. Nickey
         ROSS Products Division
         Abbott Laboratories
      (B) STREET: 625 Cleveland Avenue
      (C) CITY: Columbus
      (D) STATE: Ohio
      (E) COUNTRY: United States of America
      (F) ZIP: 43215
   (v) COMPUTER READABLE FORM.
      (A) MEDIUM TYPE: Diskette, 3.5 inch, 1.44 Mb Storage
      (B) COMPUTER: IBM Compatible
      (C) OPERATING SYSTEM: MS-DOS Version 6.21
      (D) SOFTWARE: WordPerfect Version 6.0a
   (vi) CURRENT APPLICATION DATA:
      (A) APPUCATION NUMBER:
      (B) FILING DATE:
      (C) CLASSIFICATION:
   (vii) PRIOR APPLICATION DATA:
      (A) APPLICATION NUMBER: US/249,584
      (B) FILING DATE: 26-MAY-1994
   (ix) TELECOMMUNICATION INFORMATION:
      (A) TELEPHONE: (614) 624-7080
      (B) TELEFAX: (614) 624-3074
      (C) TELEX: None
(2) INFORMATION FOR SEQ ID NO: 1
   (i) SEQUENCE CHARACTERISTCS:
      (A) LENGTH: 31 base pairs
      (B) TYPE: Nucleic acid
      (C) STRANDEDNESS: Single
      (D) TOPOLOGY: Linear
   (ii) MOLECULE TYPE: DNA
   (xi) SEQUENCE DESCRIPTION: SEQ ID NO: 1
   CGCTGCAGCA TATGCGTGAA ACCATCGAAT C 31
(3) INFORMATION FOR SEQ ID NO: 2
   (i) SEQUENCE CHARACTERISTICS:
      (A) LENGTH: 61 base pairs
      (B) TYPE: Nucleic acid
      (C) STRANDEDNESS: Single
      (D) TOPOLOGY: Linear
   (ii) MOLECULE TYPE: DNA
   (xi) SEQUENCE DESCRIPTION: SEQ ID NO: 2
   CGGGATCCTG GTCCTCGTGT TTAACTTTTT CAACTTTCTG TTTGTATTCG GTGATCGATT C 61
(4) INFORMATION FOR SEQ ID NO: 3
   (i) SEQUENCE CHARACTERISTICS:
      (A) LENGTH: 18 base pairs
      (B) TYPE: Nucleic acid
      (C) STRANDEDNESS: Single
      (D) TOPOLOGY: Linear
   (ii) MOLECULE TYPE: DNA
   (xi) SEQUENCE DESCRIPTION: SEQ ID NO: 3
   TGTAAAACGA CGGCCAGT 18
(5) INFORMATION FOR SEQ ID NO: 4
   (i) SEQUENCE CHARACTERISTICS:
      (A) LENGTH: 21 base pairs
      (B) TYPE: Nucleic acid
      (C) STRANDEDNESS: Single
      (D) TOPOLOGY: Linear
   (ii) MOLECULE TYPE: DNA
   (xi) SEQUENCE DESCRIPTION: SEQ ID NO: 4
   GGGGATCCGT ACGCGTGAAA C 21

## Description

### TECHNICAL FIELD

This invention relates to a product comprising human β-casein having 3-5 phosphate group useful for inhibiting the attachment of *Haemophilus influenzae (H. influenzae)* bacilli to human nasopharyngeal cells. More particularly, the invention relates to the use of a human β-casein for manufacturing a medicament for treating and preventing otitis media by inhibiting the attachment of *H. influenzae* to human cells characterized in that said human β-casein consists of phosphorylated recombinant human beta casein containing 3 to 5 phosphate groups.

### BACKGROUND OF THE INVENTION

Aniansson et al., Microb. Pathogenesis 1990, 8:318-323, disclose that native human milk inhibits attachment of H. influenzae to human nasopharyngeal cells. Such document though states that the structural basis for the inhibition of *H. influenzae* adherence by milk is yet to be defined. As further background art, WO 91/08675 teaches the use of the non-phosphorylated form of recombinant human β-casein to solve inherent digestibility and allergenic problems associated with the use of non-human proteins in human infant compositions..

It is generally recognized that human milk is the best nutritional source for human infants. Human milk is not only an ideal source of nutrients for the developing infant, but also contains both immunoglobulins and non-immunological factors that protect the infant from infection by various organisms. Human milk is also easily digested by the infant and is less likely to cause allergic reactions than is infant formula based on bovine milk.

Human milk differs from bovine milk as well as the milk of other mammalian species in various ways. Overall protein content and the kinds of protein differ between human and bovine milk. Four major bovine caseins have been identified. Bovine milk contains 2 α-caseins plus β- and κ-casein, but human milk contains only β- and κ-casein. Additionally, the amino acid sequences of human milk protein differ from that of other mammalian milk proteins.

Efforts have been made to develop infant milk formula that have some of the advantageous properties of human milk and avoid the disadvantages associated with bovine milk based infant formulas such as allergic reactions and incomplete digestion by the infant. An intuitively desirable method to achieve this is to add to the formula some of the known constituents of human milk, including human milk proteins in their native form. The human caseins, which differ in amino acid sequence from their bovine and other mammalian counterparts, represent important substances which, if added in their native form to infant formula, would serve to enhance the nutritional value of the formula and reduce the inherent disadvantages of non-human milk proteins.

In addition to being a source of amino acids necessary for the synthesis of proteins required for the growth and development of infants, human milk is recognized as containing proteins, including casein, that have other important biological functions. β-casein is one of the most abundant milk proteins synthesized in the mammary gland. After post-translational modification in the Golgi apparatus, it is excreted as large calcium-dependent aggregates called micelles. β-casein is not a single entity, but is a heterogeneous group of phosphoproteins secreted during lactation in response to lactogenic hormones. The primary structure of human β-casein was determined by Greenberg et al. (Journal of Biological Chemistry 259:5132-5138, 1984). It was shown to be a phosphorylated protein with phosphorylation sites at specific seryl and threonyl residues located near the amino terminus. Comparison of human and bovine β-caseins showed 47% identity. The sequence of human κ-casein was determined by Brignon et al. (Federation of European Biological Societies Letters 188:48-54, 1985). Whereas β-casein is phosphorylated, κ-casein is glycosylated.

Several biological effects have been ascribed to human milk casein including: (1) enhancement of calcium absorption; (2) inhibition of angiotensin I-converting enzyme; (3) opioid agonism; (4) and immunostimulating and immunomodulating effects.

Human casein consists largely (>80%) of the β-form with a smaller amount in the κ-form (Greenberg et al., 1984). Native β-casein is a 25 kDa protein. In human milk, β-casein molecules show variable degrees of post-translational phosphorylation ranging from zero to five phosphate groups per polypeptide chain (Greenberg et al., 1984; Hansson et al., Protein Expression and Purification 4:373-381, 1993). Phosphate groups in the native protein are attached to serine and threonine residues located near the amino terminus (Greenberg et al., 1984).

Expression of exogenous genes in bacterial cells provides a useful method for producing recombinant eukaryotic proteins. However, bacteria, such as *E. coli*, are not capable of producing the post-translational modifications required by many eukaryotic proteins as they do not possess the endogenous enzymes necessary to do so. Therefore, eukaryotic proteins produced in *E. coli* lack the specific post-translational modifications which may occur within the eukaryotic cell, such as glycosylation, phosphorylation, acetylation, or amidation.

Prior to the development of appropriate cloning techniques, the phosphorylation of purified proteins by a kinase was done *in vitro* using chemical reagents. This process requires the protein substrate and the kinase enzyme to be purified and this is not efficient or cost-effective for commercial purposes. The *in vitro* process is also inefficient when it is desired to scale-up for commercialization. There is, therefore, a need to develop a method for genetically engineering microorganisms to phosphorylate a protein *in vivo*.

Canadian Patent Application No. 2,083,521 to Pawson et al. teaches a method of producing phosphorylated exogenous protein in host cells. The method of Pawson et al. requires two vectors to be introduced into a bacterial cell. One vector has a nucleotide sequence encoding an exogenous protein that is capable of being phosphorylated by the catalytic domain of a protein kinase. The other vector has a nucleotide sequence encoding the protein kinase catalytic domain. Both vectors are introduced into *E. coli* and production of the exogenous protein and the protein kinase catalytic domain is induced so that the exogenous protein is phosphorylated. The bacterial cells are then lysed and the exogenous phosphorylated protein is isolated using standard isolation techniques.

CA No. 2,083,521 does not suggest or disclose the method disclosed herein for production of a phosphorylated, recombinant protein.

The present inventors use a single vector expressing both the substrate and the kinase enzyme. The method of Pawson et al. requires the use of two vectors. The expression system disclosed herein results in specific phosphorylation of the exogenous protein as determined by antibody to phosphoserine, while the expression system of Pawson et al. results in nonspecific phosphorylation of both host proteins and exogenous proteins. This would adversely affect the growth of host bacteria in scale-up efforts for industrial applications. The present method unlike that of Pawson et al., provides for high level production of a phosphorylated, recombinant protein suitable for commercial production.

Simcox et al., Strateqies in molecular biology 7(3):68-69 (1994) constructed two *E. coli* strains that harbor a tyrosine kinase plasmid. These TK (tyrosine kinase) strains can be used for generating phosphorylated proteins when transformed 'with a plasmid containing sequences encoding a phosphorylation target domain or protein. Both *E. coli* strains carry an inducible tyrosine kinase gene. One strain, TKB1, is useful for expressing genes whose expression is directed by the T7 promoter. The system developed by Simcox et al. differs from the present invention in that it requires two constructs, i.e., a tyrosine kinase-containing plasmid and a plasmid vector containing a gene encoding a protein or domain to be phosphorylated.

In order to better understand the structure and function of human β -casein and to permit studies of factors that affect regulation of its synthesis and secretion, cDNA for this protein was cloned and sequenced (Lönnerdal et al., Federation of European Biological Societies Letters 269:153-156,1990), and human milk β-casein was produced in *Escherichia coli* and *Saccharomyces cerevisiae* (Hansson et al., 1993). Hansson et al. demonstrated that recombinant human β-casein was expressed in the yeast, S. *cerevisiae*, using the pYES 2.0 vector (Invitrogen Corp., San Diego, CA). Production levels were estimated to be approximately 10% of the production found in *E. coli.* However, recombinant, β-casein obtained from S. *cerevisiae*, a eukaryotic cell that has endogenous enzymes capable of phosphorylating proteins, was phosphorylated, but the protein produced by *E. coli*, a prokaryotic cell that lacks the ability in its native state to phosphorylate, was non-phosphorylated. Subsequently, it was shown that recombinant human casein kinase II (rhCKII) produced in and purified from E. *coli* can phosphorylate protein substrates *in vitro* (Shi et al., Proceeding of the National Academy of Sciences, USA 91:2767-2771, 1994). One specific embodiment of the method disclosed herein uses a nucleotide sequence encoding a recombinant human casein kinase II in a single construct with nucleotide sequence encoding β-casein to transform *E. coli* and produce phosphorylated β -casein. None of the prior art suggests or discloses a single vector containing a promoter followed by a nucleotide sequence encoding a protein followed by a nucleotide sequence encoding a kinase as is disclosed herein.

### SUMMARY OF THE INVENTION

According to the present invention, a human β-casein consisting of recombinant phosphorylated human β-casein having 3 to 5 phosphate groups can be used for manufacturing a medicament for treating and preventing otitis media in a human by inhibiting the attachment *of H*. *influenzae* to human cells.

The present invention provides a product comprising a therapeutically effective amount of a human β-casein characterized in that said human β-casein consists of recombinant phosphorylated human β-casein having 3 to 5 phosphate groups for inhibiting the attachment of *H. influenzae* to human cells. In a specific embodiment, such product is an infant formula. An infant formula of the invention may comprise at least one other protein from mammalian milk. In a further embodiment, an infant formula of the invention may comprise at least one vegetable protein.

The product of the invention may be a liquid nutritional product. The liquid nutritional product may be an enteral product further comprising at least one other protein from mammalian milk. The liquid nutritional product may comprise at least one vegetable protein.

The product may be used to inhibit the attachment of *H. influenzae* to human pharyngeal cells. In particular, the product of the invention may be a product for administration via a nasal passageway for inhibiting the attachment of *H. influenzae* to human nasopharyngeal cells. As a further embodiment, the product of the invention may be a throat spray formulation for inhibiting the attachment of *H. influenzae* to human nasopharyngeal cells. The above products can be used for treating and preventing otitis media.

The recombinant phosphorylated human β-casein in any of the above products can be synthesized under the direction of a plasmid comprising:
a. a promoter;
b. followed by a nucleotide sequence encoding human β-casein; and
c. followed by a nucleotide sequence encoding an enzyme that can phosphorylate human β-casein.

Recombinant phosphorylated human β-casein for use in the products of the invention can be produced by methods disclosed herein. The methods for producing a modified recombinant human β-casein in a host cell comprises preparing a single vector encoding both an exogenous protein which is human β-casein and an enzyme capable of phosphorylating the exogenous protein. In particular, such enzyme may be casein kinase. Representative of promoters useful in the method include inducible promoters such as T7, λP_{L}, λP_{R}, and Tac and constitutive promoters such as *bla* and *spa*. Representative of host cells capable of being transformed and then expressing the phosphorylated human β-casein, include but are not limited to the bacterial cells *E*. *coli K-12* and *E. coli B, Bacillus* species, *Lactobacillus* species, and *Streptococcus* species and eukaryotic cells such as yeast cells or mammalian cells.

An exogenous protein is one that originates outside the organism that is producing it. The term is sometimes used in the relevant DNA cloning literature also to refer to the recombinant protein produced by the transformed recipient organism. Alternatively, an exogenous protein produced using DNA cloning techniques may be referred to as a recombinant protein. The terms will be used interchangeably herein since the distinction is frequently not made in the literature. However, in discussing the disclosed invention the word "recombinant" will be used to refer to the protein produced by the transformed organism, and "exogenous" will be used when referring to the native, non-recombinant protein or nucleotide sequence encoding the protein.

A method for producing a phosphorylated recombinant human β-casein in a host cell comprises the steps of preparing a single vector having a promoter sequence followed by a nucleotide sequence encoding an exogenous protein which is human β-casein capable of being phosphorylated by a protein kinase, followed by a nucleotide sequence encoding a protein kinase capable of phosphorylating the exogenous protein; transforming the host cell with the vector; expressing the vector in the host cell whereby the produced protein kinase phosphorylates the produced recombinant human β-casein; and isolating the phosphorylated human β-casein.

More particularly, the present inventors have developed a novel method for producing a phosphorylated recombinant human β-casein in bacterial expression systems in which the resulting recombinant human protein has utility for the inhibition of attachment of *H. influenzae* to human cells and in the prevention and treatment of otitis media in human infants. Using a combination of two human casein kinase encoding sequences, expressing respectively the alpha and beta subunits of the kinase, they demonstrated the *in vivo* production of recombinant phosphorylated human β-casein in *E. coli*. The sequence coding for human casein kinase II was placed in tandem with the sequence coding for β-casein with the result that a significant portion of the recombinant β-casein produced in *E. coli* was phosphorylated as in human milk.

In a specific embodiment of the above method, a nucleotide sequence encoding a human casein kinase II (hCKII βα) is co-expressed in a single construct with a nucleotide sequence encoding a human β-casein in a bacterial expression system to achieve efficient *in vivo* phosphorylation of the appropriate serine and threonine residues of recombinant human β-casein. Experiments in which a nucleotide sequence encoding hCKII βα and a nucleotide sequence encoding human β-casein were co-expressed in *E. coli* using a single inducible expression vector demonstrated the ability of recombinant hCKII to phosphorylate recombinant β-casein *in vivo*. This was an unexpected, nonobvious result requiring experimentation and inventiveness. As was demonstrated by negative results obtained in early, control experiments the disclosed method showed unexpected results. The method produces useful and beneficial results which will permit the addition of beneficial human β-casein to nutritional and pharmaceutical products.

Phosphorylated β-casein produced using the above method is demonstrated to have the same bioactivity as native human β-casein as shown by its ability to inhibit adhesion of *H. influenzae* to human pharyngeal cells.

### BRIEF DESCRIPTION OF THE DRAWINGS

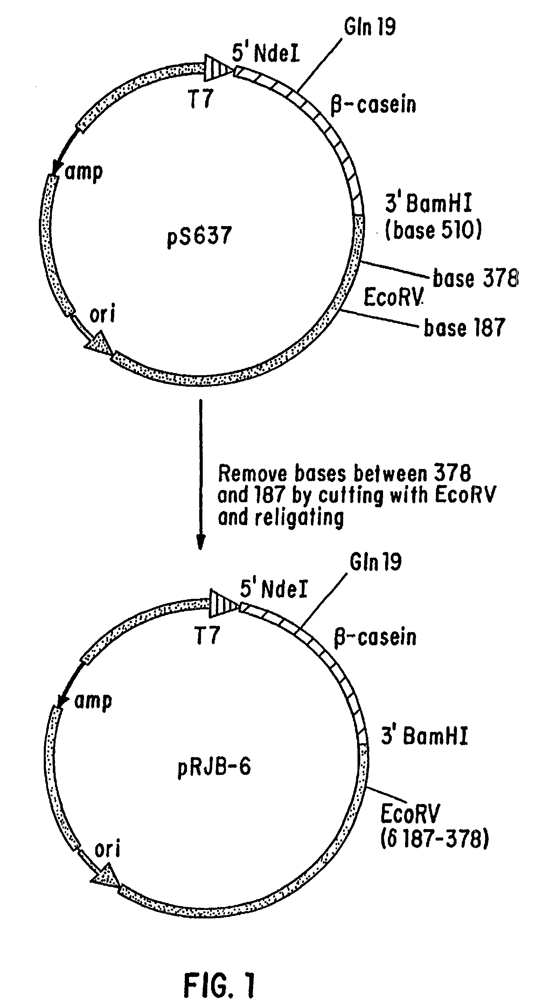

Figure 1 shows physical maps of expression vectors pS637 and pRJB-6 constructed for inducible intracellular expression in *E. coli*. 191 base pairs were removed from pS637 to produce PRJB-6.

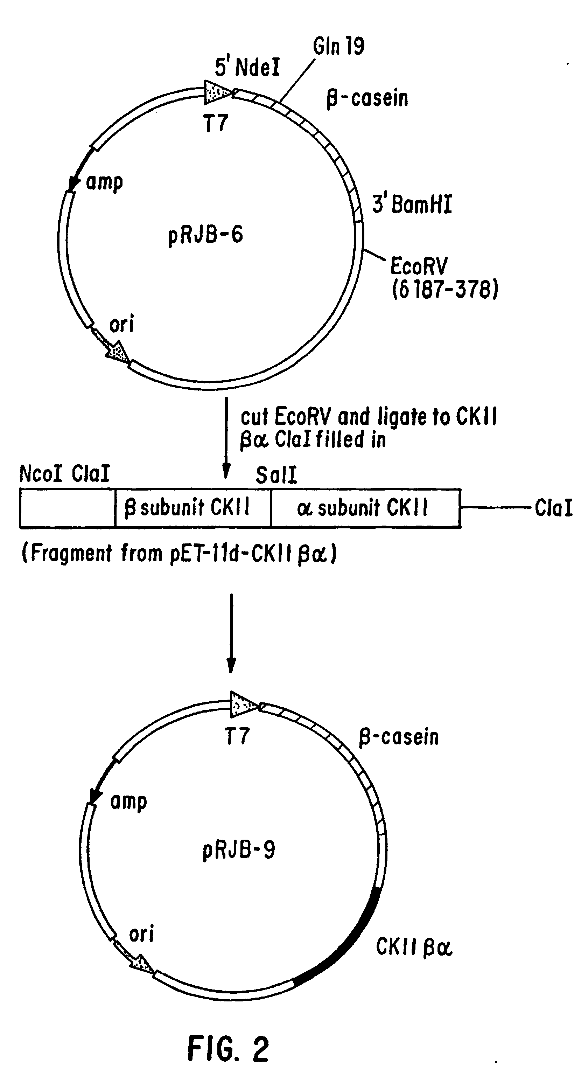

Figure 2 shows physical maps of expression vectors pRJB-6 and pRJB-9 an illustrates how pRJB-6 was cut and ligated to CKII β α to form pRJB-9.

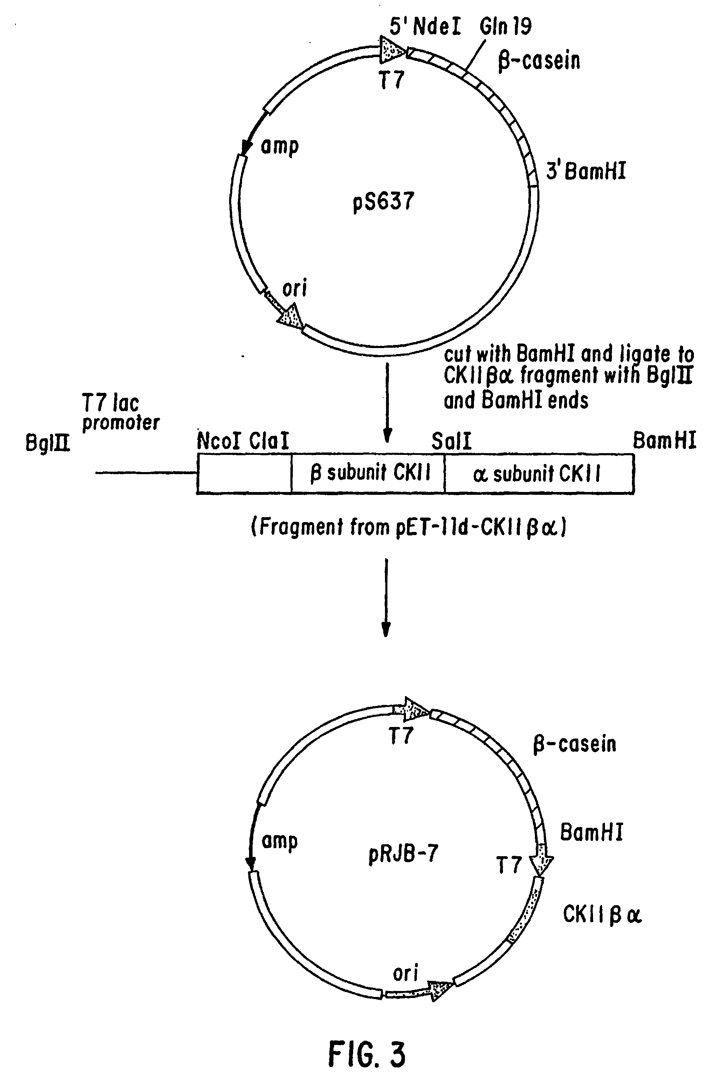

Figure 3 shows physical maps of expression vectors pS637 and pRJB-7 and shows how pS637 was cut and ligated to CKII βα to form pRJB-7. pRJB-7 has T7 promoters in front of both the β-casein and casein kinase genes.

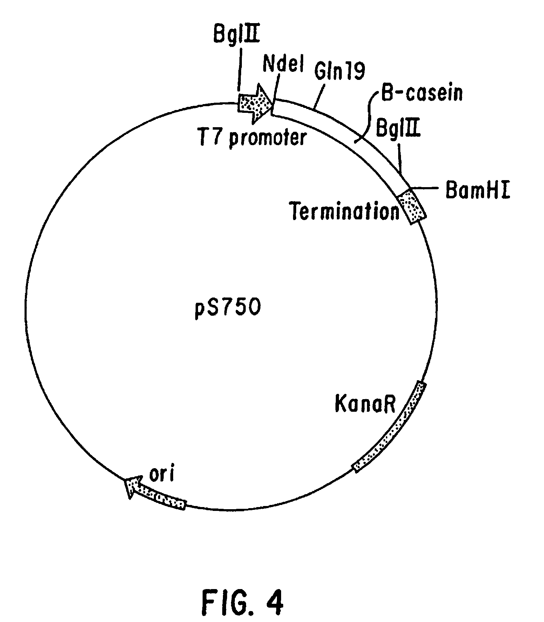

Figure 4 shows the physical map of expression vector pS750, constructed for inducible expression and to mediate production of intracellularly localized protein in *E. coli*.

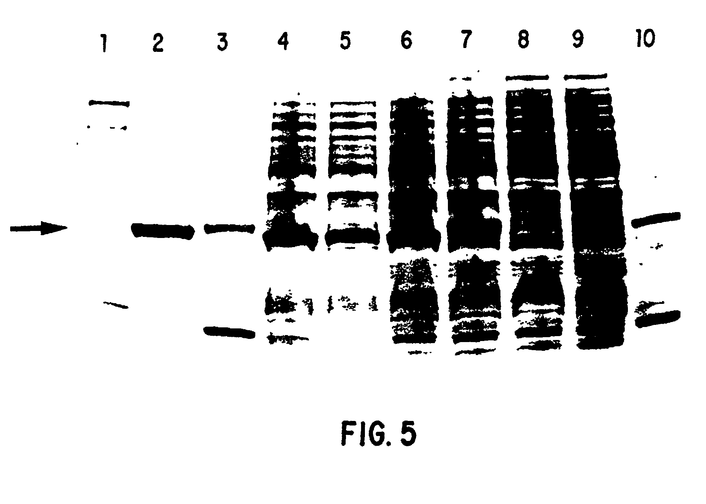

Figure 5 shows SDS-PAGE of Met-β-casein produced in *E. coli* EL21 strains and stained with Coomassie Brilliant Blue using the vectors pS750 and pET-11d-CKII βα. The codon for methionine (Met) was placed in front of the β-casein encoding sequence in the construction of plasmid pS750 because in *E. coli* and other bacteria the synthesis of their proteins begins with the amino acid methionine. This enables the ribosome to recognize the starting point for growth of a polypeptide chain. Production of intracellular recombinant β -casein is possible only when Met is inserted before the encoding sequence for the protein to be produced. Lane 1: molecular weight marker (Bio-Rad prestained, relative molecular weights 106, 80, 49.5, 32.5, 27.5, 18.5 kDa); lane 2: non-phosphorylated recombinant β-casein; lane 3: 5P-β-casein; lane 4: pS750 induced with IPTG in BL21(DE3); lane 5: pS750/pET-11d-CKII βα induced with IPTG in BL21(DE3); lane 6: pS750 induced with IPTG in BL21(DE3)pLys;S; lane 7: pS750/pET-11d-CKII βα induced with IPTG in BL21 (DE3)pLysS; lane 8: pS750 induced with IPTG in BL21(DE3)pLysE; lane 9: pS750/pET-11d-CKII βα induced with IPTG in BL21 (DE3)pLysE cells; lane 10: native β-casein with five attached phosphate groups (5P-β-casein). The arrow indicates the β-casein band.

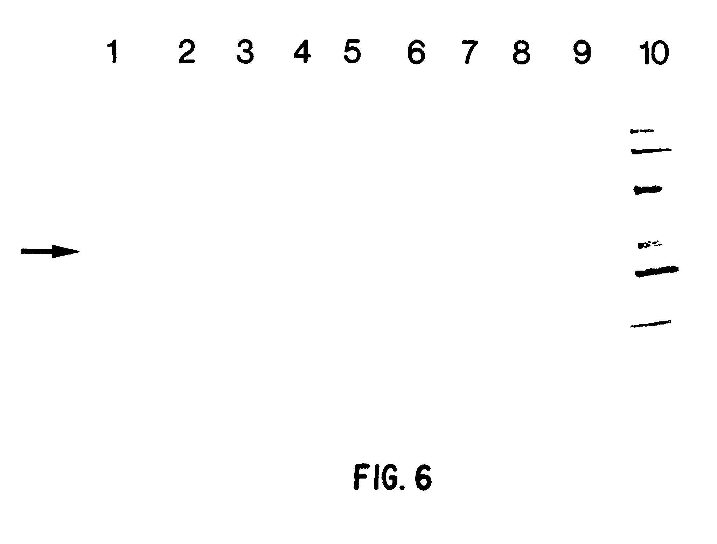

Figure 6 shows SDS-PAGE of Met-β-casein produced in *E. coli* BL21 strains stained with Ethyl Stains-All using the vectors pS750 and pET-11d-CKII βα Lane 1: native β-casein with five attached phosphate groups (5P-β-casein); lane 2: pS750/pET-11d-CKII βα induced with IPTG in BL21(DE3)pLysE cells; lane 3: pS750 induced with IPTG in BL21(DE3)pLysE; lane 4: pS75CI/pET-11d-CKII βα induced with IPTG in BL21(DE3)pLysS; lane 5: pS750 induced with IPTG in BL21(DE3)pLysS; lane 6: pS750/pET-11d-CKII βα induced with IPTG in BL21(DE3); lane 7: pS750 induced with IPTG in BL21(DE3); lane 8: SP-β-casein; lane 9: non-phosphorylated recombinant β-casein; lane 10: molecular weight marker (Bio-Rad prestained, relative molecular weights 106, 80, 49.5, 32.5, 27.5, 18.5 kDa). The arrow indicates the phosphorylated β-casein band, which is seen as a green band in the original photographs.

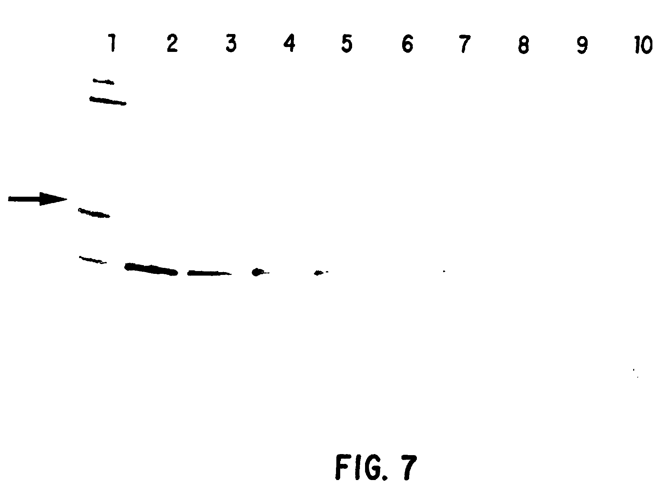

Figure 7 shows SDS-PAGE of Met-β-casein produced in *E. coli* HMS174(DE3)pLysS stained with Ethyl Stains-All using the vectors pS750 and pET-11d-CKII. Lane 1: molecular weight marker (Bio Rad prestained); lane 2: pS750 uninduced; lane 3: pS750 induced with IPTG; lane 4: pS750/pET-11d-CKII βα uninduced; lane 5: pS750/pET-11d-CKII βα induced with IPTG; lane 6: pET-11d-CKII βα uninduced; lane 7: pET-11d-CKII βα induced with IPTG; lane 8: native 5P-β-casein; lane 9 recombinant β-casein; lane 10: molecular weight marker (Bio-Rad prestained, relative molecular weights 106, 80, 49.5, 32.5, 27.5, 18.5 kDa). The arrow indicates the phosphorylated β-casein band, which is seen as a green band in the original photographs.

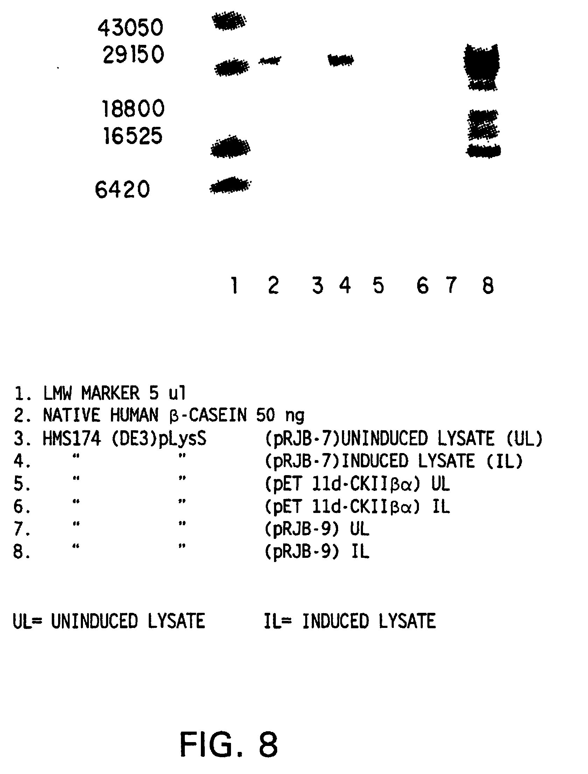

Figure 8 shows a Western immunoblot analysis using antibody to human β-casein. Lane 1: molecular weight marker (Gibco BRL, relative molecular weights 43.1, 29.2, 18.8, 16.5, 6.4 kDa); lane 2: 50 ng native human β-casein; lane 3: uninduced HMS174(DE3)pLysS(pRJB-7); lane 4: induced HMS174(DE3)pLysS(pRJB-7); lane 5: uninduced HMS174(DE3)pLysS(pET-11d-CKII βα); lane 6: induced HMS174(DE3)pLysS(pET-11d-CKII βα); lane 7: uninduced HMS174(DE3)pLysS(pRJB-9); lane 8: induced HMS174(DE3)pLysS(pRJB-9).

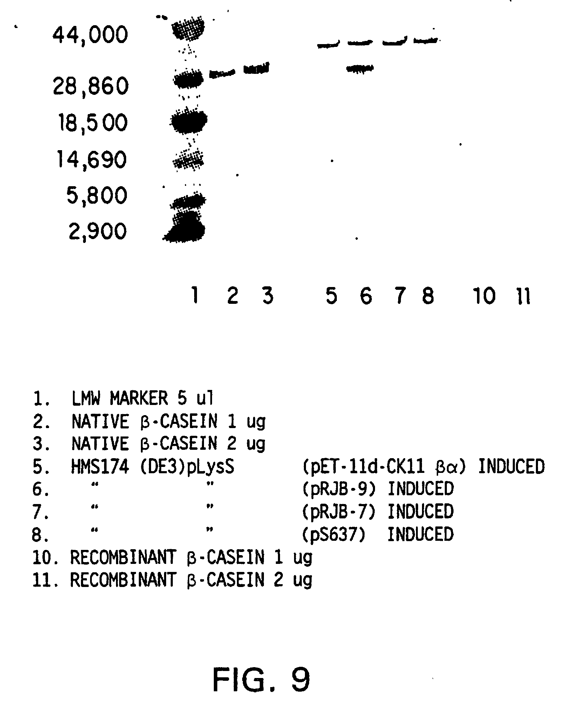

Figure 9 shows a Western immunoblot analysis with antibody to phosphoserine. Lane 1: low molecular weight marker (Gibco BRL, relative mollecular weights 44, 28.7, 18.5, 14.7, 5.8, 2.9 kDa); lane 2: 1 µg native human β-casein; lane 3: 2 µg native human β-casein; lane 5: induced HMS174(DE3)pLysS(pET-11d-CKII βα; lane 6: induced HMS174(DE3)pLysS(pRJB-9); lane 7: induced HMS174(DE3)pLysS(pRJB-7); lane 8: induced HMS174(DE3)pLysS(pS637); lane 10: 1 µg recombinant human β-casein; lane 11: 2 µg recombinant human β-casein.

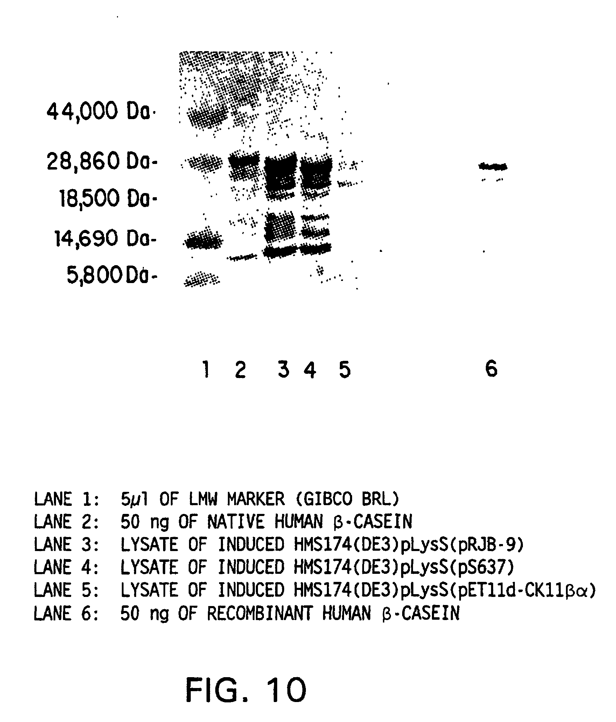

Figure 10 shows an immunoblot analysis using antibody to human β-casein. Lane 1: molecular weight marker (Gibco BRL, relative molecular weights 44, 28.9, 18.5, 14.7, 5.8 kDa); lane 2: native human β-casein; lane 3: induced HMS174(DE3)pLysS(pRJB-9); lane 4: induced HMS174(DE3)pLysS(pS637); lane 5: induced HMS174(DE3)pLysS(pET-11d-CKII βα); lane 6: recombinant human β-casein.

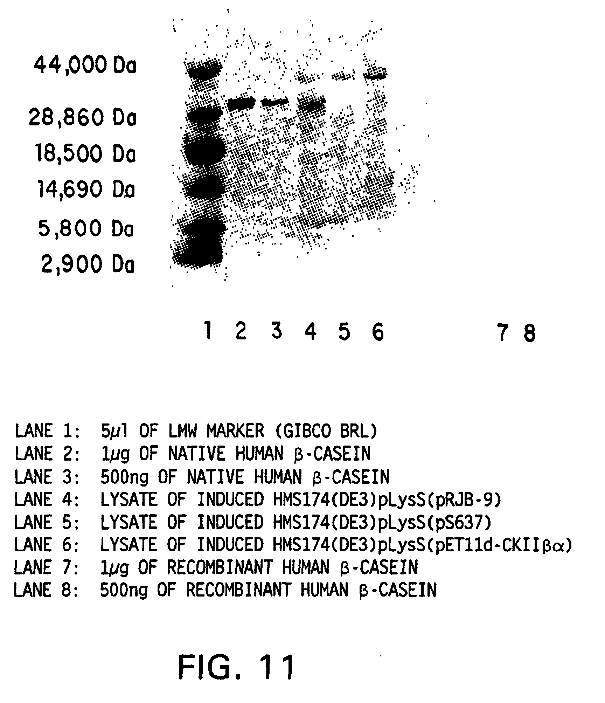

Figure 11 shows an immunoblot analysis using antibody to phosphoserine. Lane 1: molecular weight marker (Gibco BRL, relative molecular weights 44, 28.9, 18.5, 14.7, 5.8, 2.9 kDa); lane 2: 1 µg native human β-casein; lane 3: 500 ng native human β-casein; lane 4: induced HMS174(DE3)pLysS(pRJB-9); lane 5: induced HMS174(DE3)pLysS(pS637); lane 6: induced HMS174(DE3)pLysS(pET-11d-CKII β α); lane 7: 1µg recombinant human β-casein; lane 8: 500 ng recombinant human β-casein.

### DETAILED DESCRIPTION OF THE INVENTION

The product of the invention comprises a therapeutically effective amount of a human β-casein characterized in that said human β-casein consists of recombinant phosphorylated human β-casein having 3 to 5 phosphate groups for inhibiting the attachment of *H. influenzae* to human cells. Particular embodiments of the invention are disclosed hereinabove.

There is also disclosed herein a method for producing a phosphorylated recombinant β-casein in a host cell for use in the product of the invention. In a more specific embodiment the invention relates to a method for producing a phosphorylated human β-casein in a bacterial cell. The method comprises the steps of preparing a single vector having both a nucleotide sequence encoding an exogenous protein which is human β-casein that is capable of being phosphorylated by a protein kinase and a nucleotide sequence encoding an appropriate protein kinase, expressing the vector in a host cell whereby the produced kinase phosphorylates the produced exogenous protein, and isolating the phosphorylated recombinant β-casein. The present inventors have made the unexpected discovery that placing the nucleotide sequence encoding the β-casein to be phosphorylated and the nucleotide sequence encoding the kinase in tandem in a single construct with a promoter results in high level and specific phosphorylation while eliminating the negative features associated with multiple vectors such as the need for antibiotic resistance genes to be used as markers. Use of the single construct system facilitates scaling up the procedure for industrial use. It is contemplated that the method will be useful in any host cell system that is capable of expressing the exogenous protein. Suitable host cells include both prokaryotes such as bacteria and eukaryotes such as yeast and animal cells.

In the practice of the method the preferred host cell is *E. coli*, Nucleotide sequences encoding β-casein, in several different expression formats, were evaluated for expression of recombinant human, β-casein in an *E. coli* strain. After a series of experiments, it was determined that recombinant human β-casein was efficiently phosphorylated when sequences encoding human β-casein were placed in a single construct with sequences encoding human casein kinase CKII β α. Efficiency of phosphorylation was not compromised when both genes were placed in tandem in one plasmid when compared with experimental systems in which sequences encoding the kinase and the β-casein were placed in two separate vectors.

### Materials and Methods

The following materials and methods were used in the investigations described in Examples 1 to 5. Additional materials and/or methods are described for individual experiments when required. Materials and methods used in Example 6 are separately described.

### Plasmids

Plasmid construct pS637 shown in Figure 1 is identical to pS26, constructed and described in Hansson et al., (1993), which is herein incorporated by reference, except that it encodes, an additional amino acid, glutamine (Gln), at position 19. The original expression vector, pS26, was modified to create pS637 which produces a recombinant β-casein protein identical to the most abundant variant found in human populations.

The construct pS637 was prepared for co-expression with the nucleotide sequence encoding casein kinase II (Shi et al., 1994), which is hereby incorporated by reference, by placing the nucleotide sequence encoding CKII βα, which codes for two casein kinase subuhits, β and α, as a cassette, downstream from the nucleotide sequence encoding β-casein. A three-cistron tandem expression vector pET-11d-CKII βα is a plasmid containing CKII βα that was generated by Shi et al.(1994). First, pS637 was cut at two sites downstream of the β-casein encoding sequence and religated. A plasmid, pRJB-6, shown in Figure 1, was isolated which had lost 191 bases between the two cut sites. The kinase CKII βα was prepared for insertion into pRJB-6. After insertion the resulting construct was designated pRJB-9, which is shown in Figure 2. pRJB-9 is a single construct designed to mediate production of phosphbrylated β-casein. pS637 was also modified to construct the plasmids pS750 and pRJB-7 which will be described in further detail below.

### Host Cells

In the specific embodiment described below the host organism transformed by the described vectors was *E. coli*. *Other* representative organisms that could be used with the method include *Bacillus, Lactobacillus*, and *Streptococcus* species.

### Promoter

In the specific embodiment described below the T7 promoter was used. Other representative promoters that could be used with the method include the inducible promoters λP_{L} and λP_{R} and Tac and the constitutive promoters *bla* and *spa*.

### Construction of Plasmids for Bacterial Expression: Detailed Methods

### Expression vector pS637

Expression vector pS637 differs from pS26, described in Hansson et al. (1993) as it contains a nucleotide triplet encoding the glutamine (Gln) amino acid residue at position 19 of the β-casein encoding sequence. This nucleotide sequence was isolated from a human cDNA variant that is more commonly found in human populations than is the sequence of pS26. Two synthetic oligonucleotides were synthesized for polymerase chain reaction (PCR) amplification. The synthetic oligonucleotides provide convenient restriction sites and incorporated codons for amino acids used preferentially by bacteria. The two oligonucleotides were designated SYM4174 (Seq.lD NO: 1) and SYM4175 (Seq.ID NO: 2) and have the following sequences:

PCR amplification was performed as described in Ausubel et al., (eds.) Current Protocols in Molecular Biology, Vol.2, Supp. 16, 15.0.3-15.1.17 (1991) and the amplified fragment was digested with *Pst*I and *Avail* to generate an 85 bp fragment. Plasmid pS21, described in Hansson et al. (1993) was digested with *Eco*RV and *Acc*I. and a 328 bp fragment was isolated by gel electrophoresis. The isolated fragment was purified from the agarose gel by electroelution and digested with *Ava*II. This resulted in a 197 bp *Ava*II/*Acc*I fragment which was isolated. The 85 bp *Pst*I/*Ava* II digested PCR-amplified fragment and the 197 bp *Ava*II/*Acc*I were ligated into *Pst*I/*Acc*I digested pS25, a plasmid described in Hansson et al. The resulting plasmid construct as sequenced and designated pS636. A 644 bp *Nde*I and *Bam*Hl restriction fragment was isolated from pS636 and introduced into *Ndel*/*Bam*Hl digested vector pS26, a plasmid described in Hansson et al. The resulting expression vector was designated

### Expression vector pRJB-9

The pET-11d-CKII βα plasmid comprising the CKII βα encoding sequences generated by Shi et al. (1994) was prepared for co-expression with recombinant, β-casein. First, 191 base pairs (bp) were removed from pS637 by cutting two *Eco*RI sites downstream from the β-casein encoding sequence and religating pS637. A plasmid, pRJB-6 (Figure 1), was isolated, which had lost the 191 bp between the two sites and had retained a single *Eco*RV site located 132 bases away from the 3' end of the β-casein encoding sequence. The plasmid pET-11d-CKII βα, containing the CKII βα, encoding sequence, was cut with *C1a*I *and* the site was filled in with Klenow enzyme (Stratagene, CA) to create blunt ends. The filled in, *Cla*I cut CKII encoding sequence was inserted into pRJB-6, downstream from the β-casein encoding sequence, and the resulting construct was designated pRJB-9 and is shown in Figure 2.

### Expression vector pRJB-7

The construct pS637 was prepared for co-expression of recombinant β -asein and the CKII βα kinase by placing the CKII βα encoding sequence immediately after the β-casein encoding sequence. The CKII βα encoding sequence was placed as a *Bglll*/*Bam* I fragment into the *BamH* I site of pS637 and designated pRJB-7. This fragment contained the T7 promoter from its original vector, pET-11D-CKII βα. Thus, as shown in Figure 3, pRJB-7 contains two T7 promoters, one before the β-casein encoding sequence and one before the CKII βα encoding sequence.

### Expression vector pS750

To change the selective marker from ampicillin resistance to kanamycin resistance, the plasmid pS637 was digested with *Pvu*I and treated with T4 DNA polymerase to generate blunt ends. The linearized vector was; isolated and ligated with a *Hinc*II kanamycin resistance genblock (Pharmacia, Uppsala, Sweden). The resulting expression vector was designated pS750 (Figure 4).

### Expression vector for recombinant human casein kinase II

The expression vector pET-11d-CKII βα (Shi et al., 1994) was provided by Dr. C. Walsh of the Harvard Medical School, Boston, MA.

Expression experiments were carried out as described by Studier et al. (Methods in Enzymology 185:60-89, 1990). Bacteria were grown in Luria Broth (LB medium) containing 50µg/ml carbenicillin for pET-11d-CKII βα the plasmid that contains a gene conferring resistance to carbenicillin, and 50 µg/ml kanamycin for the vector pS750, a plasmid containing a gene conferring resistance to kanamycin. The medium was supplemented with 30 µg/ml chloramphenicol when the strains containing the pLys plasmids, which confer resistance to chloramphenicol, were used. For induction of the T7 expression system, the cultures were grown to a density of approximately OD₆₀₀=0.5, and then 0.4 mM isopropyl β-D-thiogalactopyranoside (IPTG) was added. The cells were harvested about 90 minutes after induction.

### Electrophoresis and Detection of Recombinant β-Casein

Cells were pelleted by centrifugation and the pellet from 1 ml of culture was dissolved in 100 µl of sample buffer, which contains Tris, glycerol, SDS, dithiothreotol (DTT), and bromophenol blue. The proteins were separated by SDS-PAGE as described in Laemmli (Nature 227:680-685, 1970). Gradient gels were cast and run in the discontinuous buffer system in a Protean (Bio-Rad, Richmond, CA) electrophoresis unit. Gels were stained as described in Laemmli. Immunoblotting was performed according to the specifications of the manufacturer (Bio-Rad).

### Procedure for isolation of modified protein

The modified protein can be isolated by any standard procedure known to those skilled in the art. Representative of such standard procedures is the following:

Cells are harvested and ruptured by standard mechanical or chemical procedures. Cells are then suspended in buffer, homogenized and centrifuged and the supernatant is discarded. The resulting insoluble pellet is resuspended and the supernatant is discarded. This results in a washed insoluble pellet that is suspended in 50 mM Tris and 6 M Urea at pH 8.2 and homogenized. β-casein supernatant I is removed resulting in an insoluble extract that is again suspended in 50 mM Tris and 6 M Urea at pH 8.2 and homogenized. β-casein supernatant II is removed and supernatants I and II are pooled. The remaining insoluble extract is discarded. The pooled supernatants are diluted 1:1 with 50 mM Tris and pH 8.2 and treated with 3 M Urea to extract β-casein. The final β-casein solution is obtained by dialyzing the Urea extract of β-casein against 50 mM ethanolamine and 100 mM NaCI at pH 9.5. centrifuging, and diluting in 50 mM ethanolamine, 100 mM NaCI at pH 9.5 to a protein concentration of 5 mg/ml. The pellet is discarded.

### EXAMPLES

The experiments described in Examples 1 and 2 show that production of recombinant β-casein is not adversely affected when bacteria are co-transformed with two vectors containing respectively a nucleotide sequence encoding β-casein and a nucleotide sequence encoding a casein kinase. They also demonstrate that recombinant phosphorylated β-casein can be produced using these two vectors in a bacterial system.

Example 3 demonstrates that the precise structure of the single plasmid was neither obvious nor expected, but that its construction required inventiveness and experimentation. Example 4 describes a system in which a single construct, containing a promoter and both the nucleotide sequence coding for the protein to be transcribed and phosphorylated and the nucleotide sequence coding for the kinase, was used to transform a bacterial strain. In Example 4, production of recombinant phosphorylated β-casein using a single plasmid was demonstrated. A single construct system for expression of extracellularly localized recombinant phosphorylated β-casein that is identical to human native β-casein is described in Example 5.

Example 6 shows a comparison of six phosphoforms of native human and recombinant human β-caseins made under the direction of the above plasmid in their ability to inhibit adhesion of the bacterium *H. influenzae* to human pharyngeal cells.

### Example 1: Production of B-casein in E.coli B: Phosphorylation of intracellularly localized recombinant Met-β-casein: BL21(DE3) strains

To analyze the ability of recombinant human CKII (rhcKll) to phosphorylate recombinant β-casein *in vivo* in a bacterial expression system, experiments were performed in *E. coli* using two inducible expression vecl.ors. The expression vector pS750 was transformed alone or in combination with expression vector pET-11d-CKII βα into the T7 host strains BL21(DE3). BL21(DE3)pLysS, and BL21(DE3)pLysE. DE3 is a DNA fragment derived from a lambda phage containing a *lac*1 repressor, a lacUV5 promoter which is inducible by isopropyl β-D-thiogalactopyranoside (IPTG), and a gene for T7 RNA polymerase. In the presence of the inducer, T7 RNA polymerase is produced resulting in transcription of the exogenous genes. Plasmid pLysS confers resistance to chloramphenicol and has little effect on growth rate and production of foreign protein. It contains a T7 lysozyme that increases stability of plasmids in *E. coli* and permits the cells to be lysed by freezing and thawing.

Results as seen in Figure 5 indicate that high levels of recombinant human Met-β-casein were produced in *E. coli* and that the amount produced was not influenced by co-production of recombinant human CKII βα. After electrophoretic separation of the proteins and phosphate staining, CKII βα is seen to have phosphorylated recombinant human Met-β-casein *in vivo*. This is shown in Figure 6 and demonstrates the ability to produce phosphorylated β-casein in a bacterial system using two vectors. This example is provided to assist in understanding the inventive nature of the method described in detail in Example 4.

### Example 2: Production of β-casein in E.coli K-12: Phosphorylation of intracellularly localized recombinant Met-β-casein: HMS174(DE3)strains

*E. coli* K-12 strains HMS174(DE3), HMS174(DE3)pLysS, and HMS 174(DE3)pLysE were evaluated as hosts for production of recombinant human Met β-casein and were transformed with pS750. The most efficient production was achieved with HMS174(DE3)pLysS. Co-expression experiments using pS750 and pET-11d-CKII βα showed strong induction of recombinant human Met-β-casein production, which was independent of the presence of pET-11d-CKII βα. Phosphate staining (Figure 7) showed efficient phosphorylatian of Met-β-casein when co-produced *in vivo* with recombinant human CKII. This example, as was also the case for Example 1, is also provided to assist in understanding the inventive nature of the method described in Example 4. A two plasmid system is inherently less desirable than the single plasmid system disclosed herein as each of the plasmids must contain an antibiotic marker so that its presence in the host cells can be monitored during the fermentation process. This necessitates the use of two antibiotics in the growth medium and retards bacterial growth.

### Example 3: Production of human β-casein E. coli K-12: Construct pRJB-7 containing both a β-casein encoding sequence and CKII βα encoding sequences: T7 Promoter in front of β-casein encoding sequence: T7 Promoter in front of CKII βα encoding sequences

The construct pRJB-7, containing the β-casein and the (CKII βα a genes each preceded by a T7 promoter, was transformed into *E. coli* K-12 host HMS174(DE3)LysS. The transformation and induction procedures followed were those of the Novagen pET system manual as described in Example 4.

### Western Blot Analysis

Separation and transfer, blocking and antibody procedures are described in Example 4. Figure 8 shows an immunoblot in which production of β-casein by *E. coli* HMS174(DE3)LysS cells containing four different constructs is compared. Lysates from both induced and uninduced cell cultures are analyzed. Cells contain pET-11d-CKII βα (plasmid with CKII β and α encoding sequences), pRJB-9 (hybrid construct with both β-casein and CKII βα encoding sequences and T7 promoter in front of β-casein encoding sequence only), or pRJB-7 (hybrid construct with both β-casein and CKII βα encoding sequences and T7 promoters in front of both β-casein and CKII βα encoding sequences). Transformation of the bacteria with pRJB-7 resulted in severe reduction of bacterial growth. *E. coli* HMS174(DE3)LysS had approximately twice the doubling time as did the same strain transformed with pRJB-9, the construct with only one T7 promoter. The Western blot shown in Figure 8 shows reduced production of recombinant β-casein by induced cells containing pRJB-7 when compared with cells containing pRJB-9. This is seen by comparing lane 4 (induced pRJB-7) with lane 8 (induced pRJB-9). Although both pRJB-7 and pRJB-9 are derived from pS637, only pRJB-9 produced amounts of β-casein equivalent to the parent construct. The presence of an additional T7 promoter before the CKII genes in the hybrid construct had the effect of both reducing cell growth and consequently reducing recombinant protein production.

Figure 9 shows a Western blot analysis in which the lysates were developed with phosphoserine antibody to detect phosphorylated protein. Induced *E. coli* HMS174(DE3)LysS cells containing pET-11d-CKII βα, pRJB-9 (hybrid construct with one T7 promoter), pRJB-7 (hybrid construct with two T7 promoters), or pS637 (contains β-casein encoding sequence but not CKII βα encoding sequence) were compared for production of phosphorylated recombinant β-casein. Phosphorylated β-casein was produced only in cells containing pRJB9 (lane 6). No phosphorylated protein was detected in lane 7, which contains the lysate of cells containing pRJB-7.

Failure to detect phosphorylated protein in the construct with two T7 promoters indicates that both inventiveness and experimentation were required in order to develop the single construct system disclosed herein for expressing an appropriately modified recombinant protein in microorganisms. Although the experiment with two T7 promoters in a single construct containing the nucleotide sequence encoding a protein and the nucleotide sequence encoding a kinase gave a negative result, under different experimental conditions the use of more than one promoter sequence should not be excluded. There may be situations where it would be favorable to use two different prompters.

### Example 4: Production of human β-casein in E. coli K-12: Construct pRJB-9 containing both β-casein encoding sequence and CKII βα encoding sequences

The method of producing recombinant human β-casein disclosed herein uses a single construct expressing both the information for transferring functional groups to specific sites and the protein to be modified. In a specific embodiment of this method the transferred functional group is phosphate. The transfer is accomplished by a kinase that is demonstrated to mediate phosphorylation of specific sites on recombinant human β-casein *in vivo*. It is demonstrated here that not only can human , β-casein be specifically phosphorylated *in vivo* by *E. coli*, but that a single-construct with a promoter located before the sequence encoding β-casein and having the advantages of a single-construct system can successfully mediate this function.

### Transformation into E. coli K-12 HMS174(DE3)pLYsS

The construct pRJB-9, containing the β-casein and CKII βα genes, was transformed into *E. coli* K-12 host HMS174(DE3)LysS. The transformation procedure followed was that of the Novagen pET system manual (4th ed., TB No.55, June, 1994).

### Induction of Expression

*E. coli* HMS174(DE3)LysS host cells containing plasmids pRJB-9 (Figure 2), pS637 (Figure 1), or pET-11d-CKII βα (Shi et al, 1994) were grown at 30°C to a density of OD₆₀₀₌0.5-0.6. Culture samples were taken before and 6 hours after adding 1 mM of the inducer IPTG. Cells from two 1 ml aliquots were pelleted by centrifugation in a microcentrifuge. Cells were resuspended in sample loading buffer for gel electrophoresis after which 500 µl of the supernatants from each aliquot were collected. The spent culture medium was concentrated in a Microcon 10 spin filter (Amicon) for 35 minutes at 10,000 x G. The retentate was collected after spinning for 3 minutes at 1,000 x G and an equal amount of sample buffer at double concentration was added.

### Western Blot Analysis

Cell lysates were separated on SDS-Polyacrylamide pre-cast Gel (Integrated Separations System) with a 10-20% gradient and transferred to an Immobilon-P membrane (Millipore, Bedford, MA) with a semi-dry blotter. Gels were electroblotted at a constant current (0.8 mA/cm²) for 45 minutes onto Immobilon PVDF filters (Millipore) using a Trans-Blot SD Transfer Cell (BioRad) The transfer buffer contained 48 mM Tris, 39 mM glycine, 1.3 mM SDS (sodium dodecyl sulfate) and 20% methanol. Prior to transfer, the filter was soaked first in methanol and then in transfer buffer. For Western blot analysis, the membrane was blocked in 3% bovine serum albumin and 0.2% Tween in TBS (25 mM Tris, 0.154 M NaCI, pH 7.4). Primary antibody to β-casein and alkaline phosphatase goat anti-rabbit antibody, the secondary antibody, were diluted 1:8000 in the blocking buffer. An additional antibody was used to detect phosphoserines. Blocking and antibody reactions were done at 25-26°C in 2% gelatin containing amplification grade porcine skin (U.S. Biochemicals) in TBS for 2 hours. The blot was then rinsed with TBS for 30 minutes. Primary antibody, mouse monoclonal anti-phosphoserine (Sigma) was diluted 1:200 or 1:100 in the 2% gelatin blocker and incubated for two hours. The blot was rinsed twice in TBS for 5 minutes. The secondary antibody, goat anti-mouse alkaline phosphatase (Sigma), was diluted 1:4,000 in the gelatin blocker, incubated for one hour, and rinsed as before in TBS. Nitro blue tetrazolium and 5-bromo-4-chloro-3-indoyl phosphate were used as substrate for color development.

Figure 10 shows an immunoblot in which production of β-casein by E. *coli* K-12 HMS174(DE3)LysS cells containing three different constructs is compared. Cells contain pS637 (plasmid with β-casein encoding sequence), pET-11d-CKII βα (plasmid with CKII β and α encoding sequences), or pRJB-9 (hybrid construct with both β-casein and CKII βα encoding sequences). Comparison of lanes 3 and 4 shows that the hybrid construct, pRJB-9, is producing equivalent amounts of β-casein to pS637, from which it was derived and which does not contain the CKII βα encoding sequences. Both pRJB-9 and pS637 produced between 400-500 mg/L of β-casein in this host cell. This experiment shows that placing the β-casein encoding sequence in tandem with the encoding sequence for CKII βα does not significantly change production of β-casein.

Figure 11 shows a Western blot analysis in which the lysates were developed with phosphoserine antibody to detect phosphorylated protein. Increased quantities-of native human β-casein and non-phosphorylated recombinant β-casein were tested in addition to the lysates of Figure 8. No phosphorylation of bacterial proteins is seen in lane 6, which contains the lysate from the CKII βα plasmid, showing that phosphorylation is specific. The cell lysate in lane 4, containing pRJB-9 with the, β-casein and CKII βα encoding sequences in tandem, shows a strong band cross-reacting with the antibody. The band of lane 4 has the same molecular weight as native human milk β-casein by electrophoretic analysis as seen in lanes 2 and 3. There was no cross-reactivity to recombinant, non-phosphorylated human β-casein, either purified as in lanes 7 and 8 or as expressed *in vivo* by pS637 in lane 5. This experiment demonstrates specific, high-level phosporylation of intact, recombinant human β-casein in *E. coli* K-12 in a bacterial system using a single construct.

### Example 5: Production of β-casein in E.coli K-12: Phosphorylation of extracellularly localized recombinant β-casein: Construct containing E. coli leader sequence, promoter. β-casein encoding sequence. pDET-11d-CKII βα

In this example there is disclosed the construction of a single plasmid that is used to transform *E. coli* K-12 and mediate production of extracellularly localized phosphorylated β-casein. To create a single construct designed for secretion of phosphorylated protein to the periplasmic space of a bacterial cell, the β-casein encoding sequence is put into an expression vector containing a leader sequence that directs protein transport to the periplasm. A polymerase chain reaction (PCR) is performed using the clone resulting from these procedures as the target DNA. The following primers synthesized at Midland Certified Reagent Co. (Midland, TX) can be used in the PCR, RO-4: 5'-TGT AAA ACG GCC ACT-3' (Seq.lD No: 3) and RO-29: 5'-GGG GAT CCG TAC GCG TGA AA C-3' (Seq.lD No: 4) The base unclerlined in RO-29 incorporates a single base change to create an *Mlul* site at the end of the β-casein encoding sequence in order to eliminate the bacterial initiation codon, methionine, for protein synthesis. This is done so that the resulting protein will have an amino acid sequence identical to that of human β-casein. The PCR fragment is then purified. The 3' end of the encoding sequence, which is not modified, is cut with *BamH* I. This fragment, containing a 5' blunt end and 3' *BamH* I end, is cloned in the expression vector pET-26b (Novagen, Madison, Wl), which contains a T7 promoter, and cut at the blunt end with *MscI* and with *BamH* I. The construct described here contains the T7 promoter, but other promoter sequences could be used. The CKII βα encoding sequence is inserted as described above for pRJB-9. Expression is induced and Western blot analysis is performed according to the procedures described in Example 4.

A Western blot is performed to identify a protein, isolated from the periplasmic space of the bacterial cells, that cross-reacts with antibody to phosphoserine and migrates similarly to native β-casein. This experiment demonstrates phosphorylation of recombinant human β-casein encoded by a sequence fused to a heterologous translational start and signal sequence, this sequence being preceded by a promoter sequence, and the sequence to be phosphorylated being located in a plasmid containing a kinase encoding sequence such as CKII βα. Production of extracellularly localized phosphorylated protein has not been previously disclosed either in a one-vector or a two-vector system.

The advantage of extracellular over intracellular localization ol the produced phosphorylated protein lies in the ease of its purification. The periplasmic space of bacterial cells contains less extraneous matter than the interior of the cell so that isolation of the purified protein is expedited. This is particularly advantageous during commercial production.

### Example 6:Comparison of Anti-Adhesion Bioactivity of Native and Recombinant Human β-Casein

*Haemophilus* are small, gram-negative bacilli with a lipopolysaccharide-protein cell wall and are obligate parasites present on the mucous membranes of humans and animal species. The surface of many but not all strains of *Haemophilus influenzae* is covered with a polysaccharide capsule. Nonencapsulated, nontypeable *H. influenzae* strains colonize the upper respiratory tract in most individuals within the first few months of life and is the species most commonly associated with several diseases including otitis media and sinusitis. (Murray et al., Medical Microbiology, 2d ed., p.260, 1994). They can also exacerbate chronic bronchitis.

An assay was performed to compare the activity of native human β-casein with recombinant human β-casein synthesized in cells contaiining pRJB-9 in inhibiting adherence of *H. influenzae* to human pharynx cellls. Comparisons were made between proteins phosphorylated with 0 to 5 phosphates.

### Cells and bacterial strains

Detroit 562 human pharynx carcinoma cells (DT 562) were obtained from the American Type Culture Collection (Rockville, MD). The *H. influenzae* nontypeable bacterial strain was obtained from Dr. Lauren Bakaletz at the Ohio State University.

### Cell culture

DT 562 cells seeded into 96-well plates (Costar, Cambridge, MA) at a density of 20,000-25,000 cells per well were cultured in Dulbeccos's Modlified Eagle Medium (GIBCO, Grand Island, NY) supplemented with 10% fetal bovine serum (FBS) (Hyclone, Logan, UT). Cells were incubated in a humidified atmosphere of 95% air and 5% carbon dioxide at 37°C. Experiments were conducted when cells were at least 90% confluent. Plates containing cells were washed three times with 200 µL of Hanks Balanced Salt Solution (HBSS) (GIBCO) to remove serum proteins before the addition of bacteria.

### Native human β-casein

β-casein isolated from human milk was purchased from Symbicom AB, P.O. Box 1451, S-902 24 Umea, Sweden.

### Separation of Phosphoforms

Cells were harvested by centrifugation at 7000 x 9 for 10 minutes at 40°C. Supernatant was removed and the pelleted cells were subjected to the freeze/thaw method described in Johnson et al. (Bio/Technology, December 12, 1994, pp.1357-1360) to release the recombinant β-casein. After filtration through a 0.45 µ membrane, samples containing β-casein were loaded onto an anion exchange column (Mono Q 10/10, Pharmacia Biotech, Uppsala, Sweden). Various phosphoforms were resolved on a linear gradient of 0 to 0.5 M NaCI in 20 mM ethanolamine, 6 M urea, at pH 9.5 over a period of 50 minutes.

Different phosphoforms of recombinant β-casein were identified by comparison of their elution times with those of purified native human milk β-casein.

### Radiolabeling of bacteria

*H. influenzae* were streaked onto chocolate agar plates from frozen aliquots of a low passage number and incubated at 37°C in a humidified atmosphere of 95% air and 5% carbon dioxide for 18 hours to obtain log phase cultures. Bacteria harvested in phosphate buffered saline (PBS) supplemented with 0.05% bovine serum albumin (BSA) were centrifuged and resuspended in a volume of PBS/BSA yielding an optical density of 2.4 at a wave length of 600 nm (OD₆₀₀). ¹¹¹Indium-oxine (¹¹¹In) (Amersham, Arlington Heights, IL) was used to radiolabel the. bacteria. 50µCi of the ¹¹¹In solution was added to 2.5 ml of the bacterial suspension and incubated for 20 minutes at 37°C. The radiolabeled bacteria were washed twice with 10 ml HBSS to removed unbound ¹¹¹In and resusupended in 5 ml HBSS supplemented with 30 nM HEPES buffer (N-2hydroxyethylpeperazine-N'-2-ethane sulfonic acid). 25 µL of the ¹¹¹In labeled bacterial suspension were preincubated with 25 µL of the test agent in a polypropylene 96-well plate for 15 minutes at 37°C to allow binding of the agent to the bacteria.

### Quantitation of adhesion

25 µL of the preincubation mixture containing radiolabeled bacteria and either native human or recombinant β-casein was pipetted into each well of the assay plate containing DT 562 cells. The assay plate was incubated for 20 minutes at 37°C to allow adhesion of the bacteria to the cell monolayer. Nonadhering bacteria were removed by washing the plate three times with HBSS. The assay was terminated by the addition of 100 µL of 0.05 N sodium hydroxide to disrupt the cell monolayer and the adhering *H. influenzae*. The contents of each well was placed in a Cobra polypropylene tube and counted on a Cobra gamma counter (Packard, Meriden, CT). Results were calculated by averaging the results of four replicates. Results are presented as tlhe percent inhibition of bacterial adhesion with native human or recombinant (pRJB-9 β-casein at 6 different phosphorylation levels when compared to bacterial attachment in control wells containing no test agent.

### Results

Anti-adhesion activity is only seen consistently when β-casein is phosphorylated with 3, 4, or 5 phosphate groups. At lower levels of phosphorylation little or no anti-adhesion was observed with either native or recombinant β-casein. However, at higher phosphoforms when β-casein had 3, 4, or 5 phosphates there was essentially no difference between the anti-adhesion bioactivity of native or recombinant (pRJB-9) human β -caseins. These results show that the bioactivity of β-casein in inhibiting adhesion of *H. influenzae* to human pharyngeal cells depends upon the level of phosphorylation. Unphosphorylated or minimally phosphorylated β-casein is ineffective. Attachment of 3, 4, or 5 phosphate groups is required for inhibition of adhesion of H. *influenze* to human pharyngeal cells. Resutts also demonstrate that phosphorylated recombinant β-casein made with a plasmid as described above is as effective as native human β-casein in inhibiting adhesion of *H. influenzae*. These results are summarized in Table 1.

**TABLE 1**

| **ANTI-ADHESION BIOACTIVITY OF NATIVE AND RECOMBINANT (pRJB-9) HUMAN BETA-CASEINS** | | | | | |
|---|---|---|---|---|---|
| Native Hβ | | | Recombinant Hβ | | |
| Phosphoform | Test Concentration (mg/ml) | Adhesion Inhibition | Phosphoform | Test Concentration (mg/ml) | Adhesion Inhibition |
| 0P | 1.00 | 15% | 0P | 0.40 | -2% |
| 1P | 1.00 | 0% | 1P | 0.76 | -4% |
| 2P | 1.00 | -11% | 2P | 0.76 | 35% |
| 3P | 1.00 | 47% | 3P | 0.76 | 43% |
| 4P | 1.00 | 52% | 4P | 0.76 | 51% |
| 5P | 1.00 | 50% | 5P | 0.76 | 48% |

*H. influenzae* has been identified as a causative factor for otitis media (Murray et al., 1994). Since it has been demonstrated in the experiments described above that recombinant human β-casein phosphorylated in at least three sites under the direction of the plasmid disclosed herein inhibits adhesion of *H. influenzae* to human cells, it is concluded that phosphorylated recombinant human β-casein, as described above, may be used in the prevention and treatment of otitis media in humans, particularly in human infants.

Therapeutic effects may be provided by enterally feeding or ingesting an enteral liquid nutritional product, such as infant formula, comprising a therapeutically effective amount of phosphorylated recombinant human β-casein with 3 or more phosphate groups. Preferably the recombinant phosphorylated human β-casein used in the practice of the invention is synthesized under the direction of a plasmid consisting essentially of a promoter, followed by a nucleotide sequence, encoding human β-casein, followed by a nucleotide sequence encoding an enzyme that can phosphorylate human β-casein. It: is understood that an enteral liquid nutritional product, such as an infant formula, used in the practice of the invention may further contain at least one other protein from a mammalian milk, such as human milk, cow's milk, or goat's milk, and/or at least one other protein from a vegetable source, such as soybeans or rice. The attachment of *H. influenzae* to human oropharyngeal cells may also be inhibited by administering via a nasal passageway, or as a throat spray, a formulation containing a therapeutically effective amount of phosphorylated recombinant human β-casein. Such a nasally administered formulation may be in the form of either drops or a spray. Administration of enteral, throat spray and nasal products is believed to be effective because the interaction ol human β-casein is believed to occur by direct contact in the nasopharynx rather than after ingestion and digestion of the β-casein.

The disclosed methodology will allow commercial-scale production of phosphorylated, recombinant human β-casein in microorganisms.

Phosphorylation of β-casein in a bioreactor makes possible large-scale synthesis in a fermentor of recombinant β-casein that is equivalent to native human β-casein. This will facilitate the production of infant formula containing human β-casein in its native phbsphbrylated state.

The single plasmid system is preferable to a two-plasmid system for industrial production of fermented proteins such as recombinant, phosphorylated human β-casein. Large-scale production of recombinant protein without the selective pressure provided by antibiotics in the growth medium results in plasmid loss during the fermentation process since the cells containing the plasmids would have no selective advantage over those that contained only one or no plasmids, but would be burdened by the presence of the plasmids resulting in slower growth. However, use of multiple antibiotics to provide the selective pressure necessary to maintain both plasmids in the bacteria during fermentation frequently retards bacterial growth and results in lower yield of the desired recombinant product. Therefore, for industrial purposes the single-plasmid system disclosed herein is greatly preferable to previously disclosed two-plasmid systems.

Phosphorylated recombinant human β-casein with 3 to 5 phosphate groups can be incorporated into any standard or specialized enteral liquid nutritional product including but not limited to infant formulas containing protein from non-human mammalian milk such as bovine or goat milk or protein from vegetable sources such as soybeans or rice, as well as other beverages consumed by young children. A product incorporating phosphorylated recombinant humari β-casein having 3 to 5 phosphate groups has utility for the inhibition of attachment of *H. influenzae* to human cells and in the treatment and prevention of otitis media in human infants.

The discovery disclosed herein of a novel method for producing recombinant, phosphorylated human β-casein, with characteristics similar or identical to that of native human β-casein, makes feasible the addition of this protein to infant formula so as to render it more similar to human milk with consequential benefits to developing infants.

## Claims

1. A product comprising a therapeutically effective amount of a human β-casein **characterized in that** said human β-casein consists of recombinant phosphorylated human β-casein having 3 to 5 phosphate groups for inhibiting the attachment of *H. influenzae* to human cells.

2. The product of Claim 1 which is an infant formula.

3. The product of Claim 2 further comprising at least one other protein from mammalian milk.

4. The product of Claim 2 further comprising at least one vegetable protein.

5. The product of Claim 1 which is a liquid nutritional product.

6. The product of Claim 5 wherein the liquid nutritional product is an enteral product further comprising at least one other protein from mammalian milk.

7. The product of Claim 5 further comprising at least one vegetable protein.

8. The product of Claim 1 in which said human cells are pharyngeal cells.

9. The product of Claim 1 which is a product for administration via a nasal passageway for inhibiting the attachment of *H. influenzae* to human nasopharyngeal cells.

10. The product of Claim 1 which is a throat spray formulation for inhibiting the attachment of *H. influenzae* to human nasopharyngeal cells.

11. The product of any one of Claims 1-10 for treating and preventing otitis media.

12. The product of any of Claims 1-11 wherein the recombinant phosphorylated human β-casein is synthesized under the direction of a plasmid comprising:
a. a promoter;
b. followed by a nucleotide sequence encoding human β-casein; and
c. followed by a nucleotide sequence encoding an enzyme that can phosphorylate human β-casein.

13. The use of a human β-casein for manufacturing a medicament for treating and preventing otitis media in a human by inhibiting the attachment of H. influenzae to human cells, **characterized in that** said human β-casein consists of recombinant phosphorylated human β-casein having 3 to 5 phosphate groups.

## Patentansprüche

1. Ein Produkt, das eine therapeutisch wirksame Menge eines menschlichen β-Caseins umfaßt, **dadurch gekennzeichnet, daß** das menschliche β-Casein aus rekombinantem phosphoryliertem menschlichen β-Casein mit 3 bis 5 Phosphatgruppen besteht, zur Inhibition der Anheftung von *H. influenzae* an menschliche Zellen.

2. Das Produkt gemäß Anspruch 1, welches eine Säuglingsnahrung ist.

3. Das Produkt gemäß Anspruch 2, welches weiterhin mindestens ein weiteres Protein aus Milch von Säugetieren umfaßt.

4. Das Produkt gemäß Anspruch 2, welches weiterhin mindestens ein pflanzliches Protein umfaßt.

5. Das Produkt gemäß Anspruch 1, welches ein flüssiges Ernährungsprodukt ist.

6. Das Produkt gemäß Anspruch 5, worin das flüssige Ernährungsprodukt ein enterales Produkt ist, welches mindestens ein weiteres Protein aus Milch von Säugetieren umfaßt.

7. Das Produkt gemäß Anspruch 5, welches weiterhin mindestens ein pflanzliches Protein umfaßt.

8. Das Produkt gemäß Anspruch 1, worin die menschlichen Zellen pharyngeale Zellen sind.

9. Das Produkt gemäß Anspruch 1, welches ein Produkt zur Verabreichung über einen nasalen Weg zur Inhibition der Anheftung von *H. influenzae* an menschliche nasopharyngeale Zellen ist.

10. Das Produkt gemäß Anspruch 1, welches eine Halssprayzusammensetzung ist, zur Inhibition der Anheftung von *H. influenzae* an menschliche nasopharyngeale Zellen.

11. Das Produkt von irgendeinem der Ansprüche 1-10 zur Behandlung und Vermeidung von Otitis media.

12. Das Produkt von irgendeinem der Ansprüche 1-11, worin das rekombinante phosphorylierte menschliche β-Casein synthetisiert wird unter der Steuerung eines Plasmids, welches folgendes umfaßt:
a. einen Promoter;
b. gefolgt von einer Nukleotidsequenz, welche menschliches β-Casein kodiert; und
c. gefolgt von einer Nukleotidsequenz, welche ein Enzym kodiert, welches menschliches β-Casein phosphorylieren kann.

13. Die Verwendung eines menschlichen β-Caseins zur Herstellung eines Medikaments zur Behandlung und Vermeidung von Otitis media beim Menschen durch Inhibition der Anheftung von H. influenzae an menschliche Zellen, **dadurch gekennzeichnet, daß** das menschliche β-Casein aus rekombinantem phosphorylierten menschlichen β-Casein mit 3 bis 5 Phosphatgruppen besteht.

## Revendications

1. Produit comprenant une quantité thérapeutiquement efficace d'une β-caséine humaine, **caractérisé en ce que** ladite β-caséine humaine consiste en β-caséine humaine phosphorylée recombinée ayant 3 à 5 groupes phosphate pour inhiber la fixation de H. *influenzae à* des cellules humaines.

2. Produit selon la revendication 1 qui est une formule pour jeunes enfants.

3. Produit selon la revendication 2, comprenant en outre au moins une autre protéine de lait de mammifère.

4. Produit selon la revendication 2, comprenant en outre au moins une protéine végétale.

5. Produit selon la revendication 1 qui est un produit nutritionnel liquide.

6. Produit selon la revendication 5, où le produit nutritionnel liquide est un produit entérique comprenant en outre au moins une autre protéine de lait de mammifère.

7. Produit selon la revendication 5, comprenant en outre au moins une protéine végétale.

8. Produit selon la revendication 1, où lesdites cellules humaines sont des cellules du pharynx.

9. Produit selon la revendication 1 qui est un produit pour l'administration par une voie nasale pour inhiber la fixation de *H. influenzae* à des cellules du nasopharynx humaines.

10. Produit selon la revendication 1 qui est une formulation de spray pour la gorge pour inhiber la fixation de *H. influenzae* à des cellules du nasopharynx humaines.

11. Produit selon l'une quelconque des revendications 1 à 10 pour traiter et prévenir l'otite moyenne.

12. Produit selon l'une quelconque des revendications 1 à 11, où la β-caséine humaine phosphorylée recombinée est synthétisée sous la direction d'un plasmide comprenant :
a. un promoteur ;
b. suivi par une séquence nucléotidique codant la β-caséine humaine ; et
c. suivi par une séquence nucléotidique codant une enzyme qui peut phosphoryler la β-caséine humaine.

13. Utilisation d'une β-caséine humaine pour la fabrication d'un médicament pour traiter et prévenir l'otite moyenne chez un humain par inhibition de la fixation de *H. influenzae* à des cellules humaines, **caractérisée en ce que** ladite β-caséine humaine consiste en β-caséine humaine phosphorylée recombinée ayant 3 à 5 groupes phosphate.
